# EUROPÄISCHE PATENTSCHRIFT

(11) **EP 3 972 539 B1**
(45) Veröffentlichungstag und Bekanntmachung des Hinweises auf die Patenterteilung: **14.08.2024**
(21) Anmeldenummer: 20726064.7
(22) Anmeldetag: 13.05.2020
(51) Int. Cl.: A61F 5/01, B25J 9/00

(54) **VORRICHTUNG ZUM UNTERSTÜTZEN WENIGSTENS EINES ARMES**
DEVICE FOR SUPPORTING AT LEAST ONE ARM
DISPOSITIF DE SOUTIEN D'AU MOINS UN BRAS

(30) Priorität: 22.05.2019 DE 102019113684
(43) Veröffentlichungstag der Anmeldung: 30.03.2022
(73) Patentinhaber: Ottobock SE & Co. KGaA, 37115 Duderstadt (DE)
(72) Erfinder: SCHIRRMEISTER, Benjamin, 37073 Göttingen (DE)
(74) Vertreter: Gramm, Lins & Partner Patent- und Rechtsanwälte PartGmbB
(86) Internationale Anmeldenummer: PCT/EP2020/063295
(87) Internationale Veröffentlichungsnummer: WO 2020/234077

(56) Entgegenhaltungen:
- WO-A1-2018/073629
- CN-A- 108 500 957
- US-A- 5 865 770
- US-A1- 2014 158 839
- US-A1- 2016 339 583

## Beschreibung

Die Erfindung betrifft eine Vorrichtung zum Unterstützen wenigstens eines Armes eines Benutzers, wobei die Vorrichtung wenigstens ein Armstützelement mit einer Armschale zum Anlegen an den Arm, wenigstens einen passiven Aktuator, der eingerichtet ist, eine Kraft auf das wenigstens eine Armstützelement aufzubringen, und wenigstens ein Gegenlager für die aufzubringende Kraft aufweist, dass wenigstens ein Kraftübertragungselement und ein Gegenlagerelement aufweist, wobei das Armstützelement um wenigstens eine Schwenkachse schwenkbar über ein Gelenk mit dem Kraftübertragungselement verbunden ist.

Derartige Vorrichtungen sind beispielsweise aus der US 2016/0081871 A1 bekannt. Derartige Vorrichtungen verfügen über Scharniergelenke, die ggf. federbelastet sind, um eine Unterstützungskraft auf den Oberarm aufzubringen. Nachteilig ist jedoch, dass die verschiedenen Gelenke, die nötig sind, um die möglichen Bewegungen einer Schulter nachzubilden, sehr genau orientiert und angeordnet werden müssen, damit sich die Gelenkachsen möglichst nah an tatsächlichen Schultergelenk des Trägers schneiden.

Um dies zu beheben, ist aus der DE 10 2017 112 436 A1 eine Vorrichtung bekannt, bei der das Kraftübertragungselement des Gegenlagers am Gegenlagerelement, das beispielsweise in Form eines Hüftgurtes ausgebildet ist, nach Art eines Kugelgelenkes beweglich gelagert ist. Damit entfällt die Notwendigkeit, die Bewegungen eines Schultergelenkes durch mechanische Gelenke nachbilden zu müssen.

Es hat sich jedoch herausgestellt, dass bei derartigen Vorrichtungen die Haftung der Armschiene am Arm unzureichend sein kann, wenn der Arm lediglich in die Armschalte eingelegt wird. Dieser Effekt ist oftmals umso stärker, je weiter der Arm angehoben wird. DE 10 2017 112 436 A1 und US 2016/339583 A1 offenbaren jeweils eine Vorrichtung mit den Merkmalen des Oberbegriffs des Anspruchs 1.

Der Erfindung liegt daher die Aufgabe zugrunde, die Positionierung der Armschale am Arm des Benutzers auch bei stark angehobenen Armen zu verbessern.

Die Erfindung löst die gestellte Aufgabe durch eine Vorrichtung zum Unterstützen wenigstens eines Armes eines Benutzers gemäß dem Oberbegriff des Anspruchs 1, die sich dadurch auszeichnet, dass die Vorrichtung ein Rückhalteelement aufweist, das im angelegten Zustand der Vorrichtung ein Verschieben der Armschale entlang des Armes Richtung Hand begrenzt, auf ein Verschieben der Armschale in die entgegengesetzte Richtung jedoch keinen Einfluss hat, wobei das wenigstens eine Rückhalteelement flexibel aber unelastisch ausgebildet ist und/oder zumindest einen Abschnitt aufweist, der axillar verläuft.

Der Erfindung liegt die Erkenntnis zugrunde, dass es insbesondere bei angehobenem Arm bei bestimmten Ausführungsformen der hier beschriebenen Vorrichtungen dazu kommen kann, dass die durch den passiven Aktuator auf das Armstützelement aufgebrachte Kraft dazu führt, dass die Armschale entlang des Armes Richtung Hand verschoben werden kann. Dies kann in Ausnahmefällen bis zu einem Verschieben der Armschale über den Ellenbogen hinaus führen. Dies wird durch das Rückhalteelement der erfindungsgemäßen Vorrichtung sicher vermieden, da ein Verschieben der Armschale entlang des Armes in Richtung Hand über einen vorbestimmten Punkt hinaus vermieden wird.

Ein derartiges Rückhalteelement ist insbesondere für die Ausgestaltungen von Vorrichtungen von Vorteil, bei denen das Kraftübertragungselement des Gegenlagers beispielsweise in Form einer Stange oder eines anderen Druckkraftübertragungselementes ausgebildet ist, an dem das Armstützelement schwenkbar angeordnet ist. Die vom passiven Aktuator auf das Armstützelement aufgebrachte Kraft wirkt dabei so auf das Armstützelement, dass ein den Arm unterstützendes Drehmoment aufgebracht wird. Dies kann beispielsweise durch einen exzentrisch angeordneten Kraftübertragungshebel oder Kraftangriffshebel erfolgen, an dem der passive Aktuator angreift. Dieser Angriffspunkt liegt dabei von der Schwenkachse des Schwenkgelenkes zwischen dem Kraftübertragungselement und dem Armstützelement entfernt, wodurch das Drehmoment erzeugt wird. Diese Ausgestaltung hat zur Folge, dass ohne eine Gegenbelastung durch den Arm, der in die Armschale eingelegt ist, der Aktuator dafür sorgt, dass das Kraftübertragungselement und das Armstützelement um die Schwenkachse des Schwenkgelenkes solange gegeneinander verschwenkt werden, bis sie parallel, also in Verlängerung zueinander, ausgerichtet sind. In diesem Zustand ist der Abstand zwischen beispielsweise dem Gegenlagerelement des Gegenlagers und der Armschale des Armstützelementes maximal.

Hebt nun der Benutzer der Vorrichtung den Arm an, vergrößert sich der zwischen dem Armstützelement und dem Kraftübertragungselement eingeschlossene Winkel. Durch die Tendenz des Aktuators, das Kraftübertragungselement und das Armstützelement parallel zueinander auszurichten, kann es daher insbesondere bei stark angehobenem Arm des Benutzers dazu kommen, dass die Armschale, um diese Streckung des Gelenkes hervorzurufen, in Richtung auf die Hand parallel zum Arm verschoben wird. Dies führt zu Störgefühlen, beeinträchtigt gegebenenfalls die Bewegungsfreiheit des Ellenbogens und Unterarmes und schmälert die Akzeptanz der Vorrichtung.

Der Erfindung liegt die Erkenntnis zugrunde, dass dieses Verhalten dadurch behoben werden kann, dass ein Rückhalteelement vorhanden ist, das diese Verschiebung verhindert oder zumindest begrenzt, auf eine Verschiebung in die entgegengesetzte Richtung jedoch keinen Einfluss hat.

Das wenigstens eine Rückhalteelement kann flexibel, aber unelastisch ausgebildet sein. Dadurch wird erreicht, dass es sich der Bewegung der Armschale, in der sich der Arm des Benutzers befindet, anpassen und ihr folgen kann, das Verschieben der Armschale in Richtung Hand, was eine Verlängerung des Rückhalteelementes zur Folge hätte, jedoch verhindert. Dabei ist es nicht notwendig, dass die Bewegung vollständig unterbunden wird. Dabei wird unter einem unelastischen Rückhalteelement insbesondere eine Ausgestaltung verstanden, bei der die beim bestimmungsgemäßen Gebrauch der Vorrichtung auftretenden Kräfte zu einer Dehnung oder Verlängerung des wenigstens einen Rückhalteelementes um höchstens fünf Zentimeter, bevorzugt höchstens drei Zentimeter, besonders bevorzugt höchstens zwei Zentimeter, höchst bevorzugt höchstens einen Zentimeter führen. Dies kann beispielsweise ermittelt werden, indem die Gesamtlänge des wenigstens einen Rückhalteelementes bestimmt wird, während der Benutzer der Vorrichtung seinen Arm beispielsweise eine vollständige Armabduktion durchführen lässt, also den Arm vom locker herunterhängenden Zustand seitlich nach oben führt, bis er vollständig nach oben gerichtet ist. An keiner Stelle innerhalb dieser Bewegung verlängert sich das wenigstens eine Rückhalteelement um mehr als den jeweils genannten Betrag, wenn das wenigstens eine Rückhalteelement unelastisch ausgebildet ist.

Alternativ oder zusätzlich dazu verfügt das wenigstens eine Rückhalteelement über zumindest einen Abschnitt, der axillar verläuft. Dies bedeutet im Sinne der vorliegenden Erfindung, das wenigstens eine Rückhalteelement im angelegten Zustand der Vorrichtung und bei herunterhängendem Arm des Benutzers oder während einer Armabduktionsbewegung in einem Bereich verläuft, der von der medialen Seite des Arms, der lateralen Seite der Brustwand seitlich begrenzt wird. Der Bereich wird nach dorsal und ventral durch die Frontalebene der Skapula, also des Schulterblattes und der Frontalebene des Sternums, also des Brustbeins, beschränkt. Die untere Grenze des Bereiches wird gebildet von einer gedachten Linie vom unteren Rippenbogen bis zum Ellenbogen. Die obere Grenze wird von der unteren Grenze der anatomischen Axilla, also der Achselhöhle, gebildet, die in der Regel als Achselfaszie (*Fascia axillaris*) definiert ist. In dieser Ausgestaltung verläuft folglich zumindest ein Abschnitt des Rückhalteelementes in diesem Bereich, wenn die Vorrichtung angelegt ist und der Benutzer den Arm hängen lässt oder eine Armabduktionsbewegung ausführt.

In einer besonders bevorzugten Ausgestaltung ist das wenigstens eine Rückhalteelement sowohl flexibel und unelastisch ausgebildet und verfügt über einen Abschnitt, der axillar verläuft.

Vorzugsweise bildet der wenigstens eine axillar verlaufende Abschnitt des wenigstens einen Rückhalteelementes mit wenigstens einem weiteren Abschnitt des Rückhalteelementes eine Schlaufe, die im angelegten Zustand der Vorrichtung einen Rumpf des Benutzers umschließt. Dabei ist vorteilhafterweise auch der weitere Abschnitt des Rückhalteelementes flexibel aber unelastisch ausgebildet. Wird beispielsweise durch den passiven Aktuator oder auf sonstige Weise eine Kraft auf das Armstützelement und insbesondere die Armschale ausgeübt, die in Richtung auf die Hand des Armes des Benutzers wirkt, verhindert die vorzugsweise unelastisch ausgebildete Schlaufe, die das wenigstens eine Rückhalteelement bildet, eine Verschiebung der Armschale entlang des Armes in Richtung Hand. Allenfalls durch die auch bei als unelastisch angesehenen Rückhalteelementen auftretende geringe Dehnung, wie sie oben definiert ist, kann eine geringfügige Verschiebung der Armschale in Richtung auf die Hand erfolgen.

Vorzugsweise ist ein zweiter axillar verlaufender Abschnitt Teil der Schlaufe. Dieser zweite axillar verlaufende Abschnitt verläuft jedoch bevorzugt in dem gegenüberliegenden Axillarbereich, also zwischen der lateralen Brustwand und dem medialen Oberarm des jeweils anderen Armes. Auch dieser zweite Axillarbereich wird durch die Frontalebene des Sternums, also des Brustbeines einerseits, die Frontalebene der Skapula, also des Schulterblattes andererseits, der gedachten Linie zwischen unterem Rippenbogen und Ellenbogen und der Untergrenze der Axillar, also der Achselfaszie, begrenzt, wenn der zweite Arm locker herunterhängt oder eine Armabduktionsbewegung ausführt.

Eine Armabduktionsbewegung ist das Anheben des Armes über die Seite.

In einer bevorzugten Ausgestaltung verfügt die Vorrichtung über ein zweites Armstützelement mit einer zweiten Armschale zum Anlegen an einen zweiten Arm des Benutzers, wobei das wenigstens eine Rückhalteelement im angelegten Zustand der Vorrichtung ein Verschieben der zweiten Armschale entlang des zweiten Armes in Richtung auf die zweite Hand begrenzt, auf ein Verschieben der zweiten Armschale in die entgegengesetzte Richtung jedoch keinen Einfluss hat. Vorzugsweise verfügt die Vorrichtung in dieser Ausgestaltung auch über einen zweiten passiven Aktuator, durch den eine unterstützende Kraft auf das zweite Armstützelement und damit auf die zweite Armschale ausgeübt werden kann, so dass auch der zweite Arm des Benutzers unterstützt wird.

Selbstverständlich ist in dieser Ausgestaltung einer Vorrichtung zum Unterstützen beider Arme mit jeweils einem Armstützelement, einem Gegenlager und einem passiven Aktuator auch eine Ausgestaltung mit wenigstens zwei Rückhalteelementen denkbar, wobei jedes der wenigstens zwei Rückhalteelemente ein Verschieben jeweils einer der Armschalen in Richtung auf die jeweilige Hand verhindert oder begrenzt. Vorzugsweise sind dabei die wenigstens zwei Rückhalteelemente flexibel aber unelastisch gemäß der oben gegebenen Definition ausgebildet und/oder verfügen über jeweils zumindest einen Abschnitt, der axillar verläuft. Dabei sind die beiden Axillarbereiche, durch die die beiden jeweils axillar verlaufenden Abschnitte verlaufen, auf den gegenüberliegenden Seiten des Rumpfes des Benutzers definiert.

Vorteilhafterweise erstreckt sich das wenigstens eine Rückhalteelement von der Armschale oder dem Armstützelement zu dem Gegenlagerelement.

Dabei hat es sich als vorteilhaft erwiesen, wenn sich der zumindest eine axillar verlaufende Abschnitt des wenigstens einen Rückhalteelementes im angelegten Zustand der Vorrichtung von oben nach unten, bevorzugt innerhalb einer Sagittalebene erstreckt.

Dies bedeutet nicht zwangsläufig, dass sich der gesamte axillar verlaufende Abschnitt in dieser Richtung erstreckt. In einer bevorzugten Ausgestaltung erstreckt sich das wenigstens eine Rückhalteelement von der Armschale oder einem anderen Teil des Armstützelementes entlang des Oberarms in Richtung Schulter und von dort von oben nach unten, vorzugsweise innerhalb einer Sagittalebene, um zum Gegenlagerelement des Gegenlagers zu gelangen.

Vorzugsweise ist das Rückhalteelement ein Kleidungsstück oder ein Teil eines Kleidungsstückes, insbesondere ein T-Shirt, ein Hemd, eine Weste oder eine Jacke.

Die Ausgestaltung als Kleidungsstück oder als Teil eines Kleidungsstückes hat den Vorteil, dass die Vorrichtung besonders leicht angelegt werden kann und keine zusätzlichen Elemente am Körper befestigt werden müssen, um beispielsweise das Rückhalteelement funktionsfähig auszugestalten. Das Rückhalteelement ist vorteilhafterweise so ausgebildet, dass es unabhängig von der Position des Armes relativ zum Rumpf des Benutzers ein Verschieben der Armschale in Richtung auf die Hand des Benutzers verhindert. Dies ist durch die Verwendung eines Kleidungsstückes auf besonders einfache Weise möglich, da das Rückhalteelement in diesem Fall automatisch in seiner richtigen Position und Orientierung und kaum relativ zum Körper des Trägers bewegbar angeordnet wird. Das Kleidungsstück ist dabei bevorzugt aus einem Material, bevorzugt aus einem Textil hergestellt, dass flexibel aber unelastisch im Sinne der vorliegenden Erfindung ist.

Vorteilhafterweise sind die Armschale und/oder das Gegenlagerelement lösbar an dem Rückhalteelement angeordnet. Dies ist insbesondere dann von Vorteil, wenn das Rückhalteelement ein Kleidungsstück ist. So kann das Rückhalteelement beispielsweise ein Stück Oberbekleidung, beispielsweise ein T-Shirt sein, das über einen Ärmel verfügt, an dem die Armschale lösbar, beispielsweise über Klettverschlussverbindungen oder Druckknopfverbindungen, angeordnet werden kann.

Alternativ oder zusätzlich dazu ist das Gegenlagerelement lösbar an dem Rückhalteelement, insbesondere in Form des Kleidungsstückes, angeordnet. Auch dies kann vorteilhafterweise über Klettverschlussverbindungen oder Druckknopfverbindungen geschehen. Zum Anlegen der Vorrichtung wird in einer besonders bevorzugten Ausgestaltung, in der sowohl das Gegenlagerelement als auch die Armschale lösbar an einem Kleidungsstück angeordnet sind, das das Rückhalteelement bildet, zunächst die Armschale und das Gegenlagerelement von dem Kleidungsstück entfernt. Dann kann das Kleidungsstück normal angelegt werden. Trägt der Benutzer der Vorrichtung das Kleidungsstück, können das Gegenlagerelement und die Armschale durch Klettverschluss oder andere Verbindungselemente am Kleidungsstück angeordnet werden. Dies ist einfach möglich und gewährleistet gleichzeitig eine Einstellbarkeit auf die individuellen Größenverhältnisse des Benutzers.

In einer bevorzugten Ausgestaltung verfügt das Kleidungsstück über eine Verstärkung, insbesondere eine Beschichtung, eine zusätzliche Textillage, insbesondere ein Gewebe, oder ein Verstärkungselement, das vorzugsweise aus einem Kunststoff ist. Dieses Verstärkungselement erstreckt sich vorteilhafterweise über zumindest einen Teil eines Weges, vorzugsweise jedoch den gesamten Weg, von der Armschale zu dem Gegenlagerelement. Auf diese Weise können die auftretenden Kräfte besonders textilschonend für das Kleidungsstück abgefangen werden und eine Verschiebung der Armschale in Richtung auf die Hand verhindert werden, ohne dass es auch bei häufigem Benutzen zu Beschädigungen des Kleidungsstückes kommt.

Vorteilhafterweise verfügt das Rückhalteelement über wenigstens einen Gurt, der an der Armschale und an einem Befestigungselement, insbesondere einem Geschirr, angeordnet ist, das im angelegten Zustand der Vorrichtung an einem Oberkörper des Benutzers angeordnet ist. Auch auf diese Weise lässt sich ein Rückhalteelement erreichen. So kann das Befestigungselement beispielsweise in Form eines Schultergurtes, Schulterhalfters oder anderem Schultergeschirr ausgebildet sein. Das Rückhalteelement ist in Form wenigstens eines Gurtes mit seinem einen Ende an diesem Befestigungselement und mit dem anderen Ende an der Armschale befestigt, sodass ein Verrutschen oder Verschieben der Armschale entlang des Arms in Richtung auf die Hand nicht möglich ist.

Vorteilhafterweise verhindert das Rückhalteelement ein Verschieben der Armschale entlang des Arms Richtung Hand soweit, dass die Armschale nicht in den Bereich des Ellenbogens gerät. Dies bedeutet insbesondere, dass der Ellenbogen nicht mit der Armschale in Berührung kommt, wenn die Vorrichtung angelegt ist.

Das wenigstens eine Rückhalteelement wirkt vorzugsweise als Gegenlager und kann so einer Kraft entgegenwirken, die direkt oder indirekt auf die Armschale in Richtung auf die Hand des unterstützten Armes wirkt. Dadurch verhindert es in bevorzugten Ausgestaltungen, dass das Gelenk, durch das das Armstützelement und das Kraftübertragungselement miteinander verbunden sind gestreckt werden kann, ohne dass der Arm bewegt, insbesondere angehoben wird. Dies kann beispielsweise dadurch geschehen, dass das wenigstens eine Rückhalteelement um einen Körperteil des Benutzers, beispielsweise den Hals oder den Rumpf, herum verläuft. Wird in diesem Fall eine Kraft auf die Armschale in Richtung auf die Hand ausgeübt, kann das wenigstens eine Rückhalteelement dieser Kraft durch eine Bewegung in die Richtung der Kraft so lange nachgeben, bis die das Körperteil umgebende Schlaufe eine weitere Bewegung des wenigstens einen Rückhalteelementes verhindert. Danach kann die Armschale nur noch durch eine Dehnung oder Verlängerung der wenigstens einen Rückhalteelementes verschoben werden. Diese ist jedoch aufgrund der Unelastizität des Materials des wenigstens einen Rückhalteelementes stark begrenzt oder gar nicht möglich.

Bevorzugt befindet sich auf einer Innenseite des wenigstens einen Rückhalteelementes eine rutschhemmende Beschichtung, beispielsweise aus einem Silikon. Dies ist insbesondere dann von Vorteil, wenn die Innenseite im angelegten Zustand der Vorrichtung mit der Haut des Benutzers in Kontakt kommt und an ihr anliegt.

Mit Hilfe der beiliegenden Figuren werden verschiedene Ausführungsbeispiele der vorliegenden Erfindung näher erläutert. Es zeigen:
- Figuren 1 bis 3 -: schematische Darstellungen einer Vorrichtung gemäß einem ersten Ausführungsbeispiel der vorliegenden Erfindung im getragenem Zustand und
- Figuren 4 bis 6 -: die Darstellungen aus den Figuren 1 bis 3 mit einer Vorrichtung gemäß einem weiteren Ausführungsbeispiel der vorliegenden Erfindung.

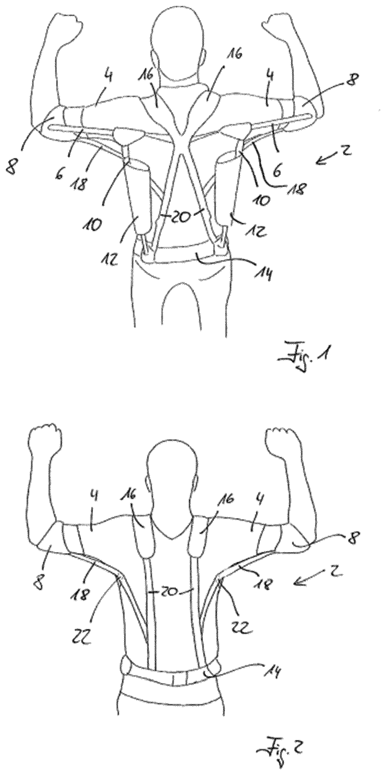

Figur 1 zeigt einen Benutzer 2 der Vorrichtung mit zwei erhobenen Armen 4 in einer Rückansicht. Die Vorrichtung verfügt über zwei Armstützelemente 6, an denen sich jeweils eine Armschale 8 befindet, die an einem Oberarm des Benutzers 2 angelegt ist. Die Armstützelemente 6 sind mit jeweils einem Kraftübertragungselement 10 verbunden, das in einer Hülse 12 verläuft, in der sich auch der in den Figuren nicht gezeigte passive Aktuator befindet, der aus Gründen der Übersichtlichkeit nicht dargestellt ist. Das untere Ende der Kraftübertragungselemente 10 ist an einem Gegenlagerelement 14 angeordnet, das in Form eines Beckengurtes oder Hüftgurtes ausgebildet ist. Über zwei Schultergurte 16, die mit dem Gegenlagerelement 14 verbunden sind, ist die Vorrichtung am Benutzer 2 festgelegt.

Die in Figur 1 gezeigte Vorrichtung verfügt zudem über zwei Rückhalteelemente 18, die mit den unteren Gurtabschnitten 20 der Schultergurte 16 verbunden sind und am gegenüberliegenden Ende an der Armschale 8 angeordnet sind. Dabei verfügt jede Armschale 8 über ein separates Rückhalteelement 18. Statt an der Armschale 8 kann das jeweilige Ende des Rückhalteelementes 18 auch an dem Armstützelement 6 angeordnet sein.

Werden die Arme 4 des Benutzers 2 aus der in Figur 1 gezeigten Position weiter angehoben, vergrößert sich der Winkel zwischen dem jeweiligen Armstützelement 6 und dem dazu gehörigen Kraftübertragungselement 10. Da der nicht dargestellte passive Aktuator eine das Armstützelement 6 unterstützende Kraft aufbringt, kann es durch den Aktuator dazu kommen, dass das Gelenk zwischen dem Armstützelement 6 und dem Kraftübertragungselement 10 gestreckt wird, ohne dass der Benutzer 2 dies wünscht. Um dies zu verhindern ist das Rückhalteelement 18 vorhanden, das verhindert, dass die Armschale 8 entlang des jeweiligen Armes 4 in Richtung auf die Hand verschoben wird. Ohne diese Verschiebung ist eine versehentliche oder unbeabsichtigte Streckung des Gelenkes nicht möglich.

Figur 2 zeigt den Benutzer 2 aus Figur 1 in einer Frontalansicht. Man erkennt die Arme 4 in gehobener Position, die Armschalen 8, die Schultergurte 16, die auch im Frontalbereich durch untere Gurtabschnitte 20 mit dem Gegenlagerelement 14 verbunden sind und die zwei Rückhalteelemente 18. Der von der Armschale 8 aus gesehene erste Abschnitt des jeweiligen Rückhalteelementes 18 verläuft axillar. In diesem Bereich ist das Rückhalteelement 18 bevorzugt als einzelner Gurt aus einem flexiblen aber unelastischen Material gebildet. Im weiteren Verlauf kommt es zu einer Gabelung 22, in der im gezeigten Ausführungsbeispiel die Rückhalteelemente 18 in einen frontalen Anteil, der in Figur 2 dargestellt ist, und einen dorsalen Anteil, der in Figur 1 dargestellt ist, aufgespalten sind.

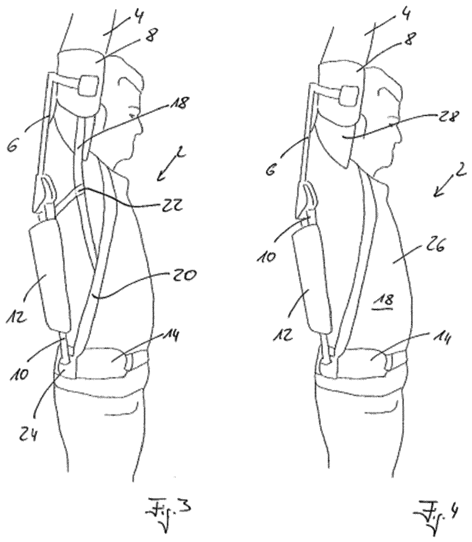

Figur 3 zeigt den Benutzer 2 in einer Seitenansicht. Der Arm 4 des Benutzers 2 ist angehoben und man erkennt die Armschale 8, die am Armstützelement 6 angeordnet ist. In der Hülse 12 verläuft sowohl das Kraftübertragungselement 10 als auch der auch in Figur 3 nicht dargestellte passive Aktuator. In der Darstellung von Figur 3 ist besonders deutlich das Rückhalteelement 18 zu erkennen, das im in Figur 3 oberen Anteil, der sich an die Armschale 8 anschließt, als ein einzelner Gurt ausgebildet ist, der an der Gabelung 22 in zwei Gurte aufgespalten wird. Sowohl der frontale Anteil als auch der dorsale Anteil sind im gezeigten Ausführungsbeispiel an unteren Gurtabschnitten 20 angeordnet und somit mit dem Gegenlagerelement 14 verbunden. In Figur 3 ist zudem deutlich zu erkennen, dass das untere Ende des Kraftübertragungselementes 10 über ein Kugelelement 24 an dem Gegenlagerelement 14 angeordnet ist. Dies kann beispielsweise dadurch erreicht werden, dass anstelle eines tatsächlichen Kugelgelenkes das Ende des Kraftübertragungselementes 10 in eine dafür vorgesehene Tasche am Gegenlagerelement 14 eingesteckt ist.

Figur 4 zeigt den Benutzer 2 in der in Figur 3 gezeigten Position mit einer Vorrichtung gemäß einem weiteren Ausführungsbeispiel der vorliegenden Erfindung. Anders als in den Figuren 1 bis 3 ist das Rückhalteelement 18 nicht in Form eines separaten Gurtes ausgebildet, sondern wird durch das T-Shirt 26 gebildet. Die Armschale 8, die an dem Armstützelement 6 angeordnet ist, ist mit dem Ärmel 28 des T-Shirts verbunden. Der auch in Figur 4 nicht gezeigte passive Aktuator, der sich entlang der Hülse 12 erstreckt, übt eine das Armstützelement 6 unterstützende Kraft aus und könnte ohne Rückhalteelement 18 dazu führen, dass die Armschale 8 entlang des Armes 4 in Richtung auf die Hand, in Figur 4 also nach oben, verschoben wird, um ein Gelenk zwischen dem Armstützelement 6 und dem Kraftübertragungselement 10 zu strecken. Da die Armschale 8 an dem T-Shirt 26, das das Rückhalteelement 18 bildet, angeordnet ist, und auch das untere Ende des T-Shirts 26 mit dem Gegenlagerelement 14 verbunden ist, kann die Armschale 8 nicht oder nur in sehr geringem Maße entlang des Armes nach oben verschoben werden, ohne dass der Arm 4 weiter angehoben wird. Dadurch wird das Verschieben der Armschale 8 entlang des Armes 4 in Richtung auf die Hand verhindert. Der Kontakt zwischen dem unteren Ende des T-Shirts 26 und dem Gegenlagerelement 14 kann beispielsweise über Klettverschluss-elemente erreicht werden. Es kann jedoch auch ausreichend sein, das Gegenlagerelement 14 in Form eines Gürtels oder Gurtes auszubilden, und auf diese Weise einen Druck auszuüben, durch den das untere Ende des T-Shirts 26 zwischen dem Gegenlagerelement 14 und dem Körper des Trägers eingeklemmt wird. Selbstverständlich ist es auch möglich, dass T-Shirt 26 beispielsweise in eine Hose einzustecken und die erforderliche Klemmwirkung über die Hose und/oder einen Gürtel der Hose zu erreichen.

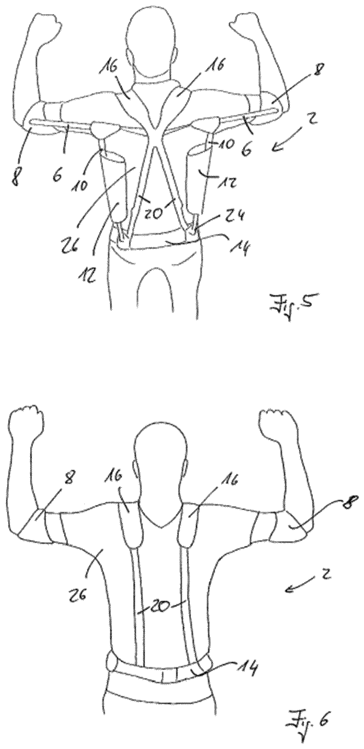

Figur 5 und 6 zeigen den Benutzer 2 in den Positionen aus den Figuren 1 und 2 mit einer Vorrichtung gemäß Figur 4. Die Armschalen 8 sind mit dem T-Shirt 26 verbunden, so dass dieses T-Shirt 26 als Rückhalteelement 18 dient. Auch hier gibt es folglich zumindest einen Abschnitt des T-Shirts 26, der axillar verläuft. Besonders bevorzugt besteht das T-Shirt 26 insbesondere in diesem Bereich aus einem Material, das zwar flexibel, aber möglichst unelastisch ausgebildet ist.

Auch in den Figuren 5 und 6 verfügt die Vorrichtung über jeweils zwei Schultergurte 16, die sowohl im Frontalbereich in Figur 6 als auch im Rückenbereich in Figur 5 über untere Gurtabschnitte 20 mit dem Gegenlagerelement 14 verbunden sind. Die Armschalen 8 befinden sich am Armstützelement 6, das über ein Gelenk mit dem jeweiligen Kraftübertragungselement 10 verbunden ist. Dieses erstreckt sich entlang der Hülse 12, in der sich auch der aus Übersichtlichkeitsgründen nicht gezeigte passive Aktuator befindet. Die unteren Enden der Kraftübertragungselemente 10 sind über Kugelgelenkes 24 mit dem Gegenlagerelement 14 verbunden.

### Bezugszeichenliste

- 2: Benutzer
- 4: Arm
- 6: Armstützelement
- 8: Armschale
- 10: Kraftübertragungselement
- 12: Hülse
- 14: Gegenlagerelement
- 16: Schultergurt
- 18: Rückhalteelement
- 20: unterer Gurtabschnitt
- 22: Kabelung
- 24: Kugelgelenk
- 26: T-Shirt
- 28: Ärmel

## Patentansprüche

1. Vorrichtung zum Unterstützen wenigstens eines Armes (4) eines Benutzers (2), wobei die Vorrichtung
- wenigstens ein Armstützelement (6) mit einer Armschale (8) zum Anlegen an den Arm (4),
- wenigstens einen passiven Aktuator,
o der eingerichtet ist, eine Kraft auf das wenigstens eine Armstützelement (6) aufzubringen, und
- wenigstens ein Gegenlager für die aufzubringende Kraft aufweist,
o das wenigstens ein Kraftübertragungselement (10) und ein Gegenlagerelement (14) aufweist,
- wobei das Armstützelement (6) um wenigstens eine Schwenkachse schwenkbar über ein Gelenk mit dem Kraftübertragungselement (10) verbunden ist,
**dadurch gekennzeichnet, dass** die Vorrichtung zusätzlich wenigstens ein Rückhalteelement (18) aufweist, das im angelegten Zustand der Vorrichtung ein Verschieben der Armschale (8) entlang des Armes (4) Richtung Hand begrenzt, auf ein Verschieben der Armschale (8) in die entgegengesetzte Richtung jedoch keinen Einfluss hat, wobei das wenigstens eine Rückhalteelement (18) flexibel aber unelastisch ausgebildet ist und/oder zumindest einen ersten Abschnitt aufweist, der eingerichtet ist, im angelegten Zustand der Vorrichtung axillar zu verlaufen.

2. Vorrichtung nach Anspruch 1, **dadurch gekennzeichnet, dass** der wenigstens eine erste verlaufende Abschnitt des wenigstens einen Rückhalteelementes (18) mit wenigstens einem weiteren Abschnitt des Rückhalteelementes (18) eine Schlaufe bildet, die im angelegten Zustand der Vorrichtung einen Rumpf des Benutzers umschließt.

3. Vorrichtung nach Anspruch 2, **dadurch gekennzeichnet, dass** ein zweiter Abschnitt, der eingerichtet ist, im angelegten Zustand der Vorrichtung axillar zu verlaufen, Teil der Schlaufe ist.

4. Vorrichtung nach Anspruch 3, **dadurch gekennzeichnet, dass** die Vorrichtung ein zweites Armstützelement (6) mit einer zweiten Armschale (8) zum Anlegen an einen zweiten Arm des Benutzers aufweist und das wenigstens eine Rückhalteelement (18) im angelegten Zustand der Vorrichtung ein Verschieben der zweiten Armschale (8) entlang des zweiten Armes Richtung der zweiten Hand begrenzt, auf ein Verschieben der zweiten Armschale (8) in die entgegengesetzte Richtung jedoch keinen Einfluss hat.

5. Vorrichtung nach einem der vorstehenden Ansprüche, **dadurch gekennzeichnet, dass** das Rückhalteelement (18) sich von der Armschale (8) oder dem Armstützelement (6) zu dem Gegenlagerelement (14) erstreckt.

6. Vorrichtung nach Anspruch 5, **dadurch gekennzeichnet, dass** sich der zumindest eine erste Abschnitt des wenigstens einen Rückhalteelementes (18) im angelegten Zustand der Vorrichtung von oben nach unten, bevorzugt innerhalb einer Sagittalebene erstreckt.

7. Vorrichtung nach einem der vorstehenden Ansprüche, **dadurch gekennzeichnet, dass** das Rückhalteelement (18) ein Kleidungsstück oder ein Teil eines Kleidungsstückes ist, wobei das Kleidungsstück vorzugsweise ein T-Shirt (26), ein Hemd, eine Weste oder eine Jacke ist.

8. Vorrichtung nach einem der vorstehenden Ansprüche, **dadurch gekennzeichnet, dass** die Armschale (8) und/oder das Gegenlagerelement (14) lösbar an dem Rückhalteelement (18) angeordnet ist.

9. Vorrichtung nach Anspruch 7 oder 8, **dadurch gekennzeichnet, dass** das Kleidungsstück eine Verstärkung aufweist.

10. Vorrichtung nach Anspruch 9, **dadurch gekennzeichnet, dass** die Verstärkung eine Beschichtung, eine zusätzliche Lage eines Textils, insbesondere eines Gewebes oder ein Verstärkungselement, insbesondere aus einem Kunststoff ist.

11. Vorrichtung nach Anspruch 9 oder 10, **dadurch gekennzeichnet, dass** sich die Verstärkung über zumindest einen Teil eines Weges, vorzugsweise den gesamten Weg, von der Armschale (8) zu dem Gegenlagerelement (14) erstreckt.

12. Vorrichtung nach einem der vorstehenden Ansprüche, **dadurch gekennzeichnet, dass** das Rückhalteelement (18) wenigstens einen Gurt aufweist, der an der Armschale (8) und an einem Befestigungselement, insbesondere einem Geschirr, angeordnet ist, das im angelegten Zustand der Vorrichtung an einem Oberkörper der Trägers angeordnet ist.

## Claims

1. A device for supporting at least one arm (4) of a user (2), wherein the device has
- at least one arm support element (6) with an arm shell (8) for placing on the arm (4),
- at least one passive actuator,
o which is configured to apply a force to the at least one arm support element (6), and
- at least one counter bearing for the force to be applied,
o which comprises at least one force transmission element (10) and a counter bearing element (14),
- wherein the arm support element (6) is connected to the force transmission element (10) via a joint such that it can be swivelled about at least one swivel axis,
**characterized in that** the device also comprises at least one restraining element (18) that restricts a displacement of the arm shell (8) along the arm (4) towards the hand when the device is mounted, but does not influence a displacement of the arm shell (8) in the opposite direction, wherein the at least one restraining element (18) is designed to be flexible yet inelastic and/or comprises at least one first section that extends in an axillary manner.

2. The device according to claim 1, **characterized in that** the at least one axillary section of the at least one restraining element (18) forms a loop with at least one further section of the restraining element (18), wherein said loop encloses a torso of the user when the device is mounted.

3. The device according to claim 2, **characterized in that** a second section, which is configured to extend in an axillary manner when the device is mounted, constitutes part of the loop.

4. The device according to claim 3, **characterized in that** the device has a second arm support element (6) with a second arm shell (8) for placing on a second arm of the user and the at least one restraining element (18) restricts a displacement of the second arm shell (8) along the second arm towards the second hand when the device is mounted, but does not influence a displacement of the second arm shell (8) in the opposite direction.

5. The device according to one of the preceding claims, **characterized in that** the restraining element (18) extends from the arm shell (8) or the arm support element (6) to the counter bearing element (14).

6. The device according to claim 5, **characterized in that** the at least one first section of the at least one restraining element (18) extends from top to bottom, preferably within a sagittal plane, when the device is mounted.

7. The device according to one of the preceding claims, **characterized in that** the restraining element (18) is an item of clothing or part of an item of clothing, the item of clothing preferably being a t-shirt (26), a shirt, a vest or a jacket.

8. The device according to one of the preceding claims, **characterized in that** the arm shell (8) and/or the counter bearing element (14) is detachably arranged on the restraining element (18).

9. The device according to claim 7 or 8, **characterized in that** the item of clothing features a reinforcement.

10. The device according to claim 9, **characterized in that** the reinforcement is a coating, an additional layer of fabric, particularly a woven fabric, or a reinforcement element, particularly made of a plastic.

11. The device according to claim 9 or 10, **characterized in that** the reinforcement extends across at least one section of a path, preferably across the entire path, from the arm shell (8) to the counter bearing element (14).

12. The device according to one of the preceding claims, **characterized in that** the restraining element (18) has at least one strap that is arranged on the arm shell (8) and an attachment element, particularly a harness, which is arranged on the user's upper body when the device is mounted.

## Revendications

1. Dispositif de support d'au moins un bras (4) d'un utilisateur (2), le dispositif comprenant
- au moins un élément de support de bras (6) ayant une coque pour bras (8) à appliquer sur le bras (4),
- au moins un actionneur passif,
• qui est conçu pour appliquer une force sur ledit au moins un élément de support de bras (6), et
- au moins un contre-appui pour la force à appliquer, qui comprend
• au moins un élément de transmission de force (10) et un élément de contre-appui (14),
- l'élément de support de bras (6) étant relié à l'élément de transmission de force (10) de manière à pouvoir pivoter autour d'au moins un axe de pivotement par l'intermédiaire d'une articulation,
**caractérisé en ce que**
le dispositif comprend en supplément au moins un élément de retenue (18) qui, à l'état appliqué du dispositif, limite un déplacement de la coque pour bras (8) le long du bras (4) en direction de la main, mais n'a aucune influence sur un déplacement de la coque pour bras (8) dans la direction opposée, ledit au moins un élément de retenue (18) étant conçu de manière flexible mais non élastique et/ou comportant au moins une première portion conçue pour être axillaire, à l'état appliqué du dispositif.

2. Dispositif selon la revendication 1,
**caractérisé en ce que** ladite au moins une première portion dudit au moins un élément de retenue (18) forme avec au moins une autre portion de l'élément de retenue (18) une boucle qui, à l'état appliqué du dispositif, entoure le torse de l'utilisateur.

3. Dispositif selon la revendication 2,
**caractérisé en ce qu'**une deuxième portion qui, à l'état appliqué du dispositif, est conçue pour être axillaire fait partie de la boucle.

4. Dispositif selon la revendication 3,
**caractérisé en ce que** le dispositif comprend un deuxième élément de support de bras (6) ayant une deuxième coque pour bras (8) destinée à être appliquée sur un deuxième bras de l'utilisateur, et ledit au moins un élément de retenue (18), à l'état appliqué du dispositif, limite un déplacement de la deuxième coque pour bras (8) le long du deuxième bras en direction de la deuxième main, mais n'a aucune influence sur un déplacement de la deuxième coque pour bras (8) dans la direction opposée.

5. Dispositif selon l'une des revendications précédentes,
**caractérisé en ce que** l'élément de retenue (18) s'étend depuis la coque pour bras (8) ou depuis l'élément de support de bras (6) jusqu'à l'élément de contre-appui (14).

6. Dispositif selon la revendication 5,
**caractérisé en ce que** ladite au moins une première portion dudit au moins un élément de retenue (18) s'étend de haut en bas, de préférence à l'intérieur d'un plan sagittal, à l'état appliqué du dispositif.

7. Dispositif selon l'une des revendications précédentes,
**caractérisé en ce que** l'élément de retenue (18) est un vêtement ou une partie d'un vêtement, le vêtement étant de préférence un T-shirt (26), une chemise, un gilet ou une veste.

8. Dispositif selon l'une des revendications précédentes,
**caractérisé en ce que** la coque pour bras (8) et/ou l'élément de contre-appui (14) est agencé de manière amovible sur l'élément de retenue (18).

9. Dispositif selon la revendication 7 ou 8,
**caractérisé en ce que** le vêtement comporte un renfort.

10. Dispositif selon la revendication 9,
**caractérisé en ce que** le renfort est un revêtement, une couche supplémentaire d'un textile, en particulier d'un tissu, ou un élément de renfort, en particulier en une matière plastique.

11. Dispositif selon la revendication 9 ou 10,
**caractérisé en ce que** le renfort s'étend sur au moins une partie d'un trajet, de préférence sur la totalité du trajet, de la coque pour bras (8) à l'élément de contre-appui (14).

12. Dispositif selon l'une des revendications précédentes,
**caractérisé en ce que** l'élément de retenue (18) comprend au moins une sangle disposée sur la coque pour bras (8) et sur un élément de fixation, en particulier un harnais, disposé sur le torse du porteur, à l'état appliqué du dispositif.
